# EUROPÄISCHE PATENTSCHRIFT

(11) **EP 1 971 328 B1**
(45) Veröffentlichungstag und Bekanntmachung des Hinweises auf die Patenterteilung: **08.06.2016**
(21) Anmeldenummer: 06829676.3
(22) Anmeldetag: 16.12.2006
(51) Int. Cl.: A61K 9/70, A61K 31/465, A61F 13/02

(54) **TRANSDERMALES THERAPEUTISCHES SYSTEM FÜR FLÜCHTIGE UND/ODER THERMOLABILE STOFFE**
TRANSDERMAL THERAPEUTIC SYSTEM FOR VOLATILE AND/OR THERMOLABILE SUBSTANCES
SYSTÈME THÉRAPEUTIQUE PERCUTANÉ POUR DES SUBSTANCES VOLATILES ET/OU THERMOLABILES

(30) Priorität: 12.01.2006 DE 102006001536; 03.06.2006 DE 102006026060
(43) Veröffentlichungstag der Anmeldung: 24.09.2008
(73) Patentinhaber: LTS LOHMANN Therapie-Systeme AG, 56626 Andernach (DE)
(72) Erfinder: JUNG, Tobias, 79588 Efringen-Kirchen (DE); HORSTMANN, Michael, 56564 Neuwied (DE); DZEKAN, Horst, 56584 Meinborn (DE)
(74) Vertreter: Siemund, Volker
(86) Internationale Anmeldenummer: PCT/EP2006/012149
(87) Internationale Veröffentlichungsnummer: WO 2007/087872

(56) Entgegenhaltungen:
- WO-A-89/07429
- WO-A-95/24172
- DE-A1- 4 332 094
- US-A- 5 462 746

## Beschreibung

Transdermales therapeutisches System für flüchtige und/oder thermolabile Stoffe Beschreibung:
Transdermale therapeutische Systeme (TTS) sind Darreichungsformen, die auf die Haut appliziert werden und dafür konzipiert sind, einen Arzneistoff nach transdermaler Aufnahme systemisch verfügbar zu machen. TTS können den therapeutischen Wert einer Arzneistoffverabreichung erhöhen, indem sie eine konstante Abgabe des Wirkstoffes über einen längeren Zeitraum in das Blutkompartiment gewährleisten. Die Vorteile dieser kontinuierlichen Wirkstoffabgabe sind in erster Linie die verlängerten Applikationsintervalle, die zu einer verbesserten Patientencompliance führen, und das pharmakokinetisch optimierte Plasmakonzentrations-Zeitprofil, das eine längere Wirkdauer bei weniger Nebenwirkungen gewährleistet. Weitere Vorteile, die in der transdermalen Applikationsroute begründet liegen, sind verbesserte gastrointestinale Verträglichkeit und eine verbesserte Bioverfügbarkeit durch Umgehung des First-Pass-Effektes.

Aufgrund all dieser Vorteile sind TTS seit einer Reihe von Jahren bekannt. Solche Systeme sind z.B. für Estradiol, Norethisteronacetat, Nicotin, Fentanyl, Tulobuterol, Ethinylestradiol/Norelgestromin, Buprenorphin oder Nitroglycerin und eine Reihe weiterer Wirkstoffe in die Therapie eingeführt. Ihr Aufbau ist in der Regel dünn und geschichtet, so dass sich mit Hilfe der direkt der Haut zugewandten Seite (Klebschicht) eine zumindest vorübergehend klebrige Verbindung zur Haut ergibt, über die der Wirkstoff abgegeben wird. TTS bestehen nach dem Stand der Technik üblicherweise aus einer arzneistoffundurchlässigen Trägerschicht (sog. Backing), einer arzneistoffhaltigen Reservoirschicht oder Matrixschicht sowie einer Haftklebeschicht zur Befestigung auf der Haut, wobei diese mit der arzneistoffhaltigen Reservoirschicht bzw. Matrixschicht identisch sein kann, und einer vor der Applikation zu entfernenden arzneistoffundurchlässigen Schutzschicht (sog. Release Liner).

Zur Verbesserung der Wirkstoffpermeation durch die Haut werden neben Polymeren, Harzen und anderen pharmazeutischen Hilfsstoffen auch bei Raumtemperatur flüssige Systemkomponenten eingesetzt, die zum Teil der Einstellung der Klebkraft, der Diffusionsverbesserung innerhalb des transdermalen therapeutischen Systems oder aber der Verbesserung der Wirkstoffpermeation durch die Haut dienen.

Sowohl Wirkstoffe wie auch vorrangig flüssige Hilfsstoffe können die bei der Herstellung störende Eigenschaft der Flüchtigkeit und/oder Thermolabilität unter Prozeßbedingungen aufweisen. Dies kann zur Folge haben, dass bei der Herstellung transdermaler Systeme, die üblicherweise im Mischen der Ausgangsstoffe in einem geeigneten organischen Lösemittel, anschließender Beschichtung in dünner Schicht auf einer Basisfolie und anschließendem, meist kontinuierlichem Trocknen bei erhöhter Temperatur besteht, deutliche Verluste eintreten.

Diese Verluste, insbesondere aus der letzten Herstellungsstufe des Trocknens, können zur Dosierungsverfälschungen und / oder Leistungsbußen der hergestellten TTS führen, die eine ordnungsgemäße Herstellung und damit eine therapeutische Anwendung insbesondere am Menschen unmöglich machen oder einschränken.

Ohne den Anspruch auf erschöpfende Aufzählung seien beispielhaft als flüchtige und / oder thermolabile Hilfsstoffe genannt:
2-Pyrrolidon, Benzylalkohol, Butanol, Butandiol und andere kurzkettige Alkohole, Cineol, Diethylenglycol, Diethylenglycolmonoethylether, Diisopropyladipat, Dodecanol, Dimethyldecylphosphoxid, Dimethylisorbid, Dimethyllauroylamid, Polydimethylsiloxan, Dimethylsulfoxid, Dodecylsulfoxid, Essigsäure, Ethylacetat und andere flüchtige aliphatische und aromatische Ester (die Gemischtbestandteile vieler etherischer Öle sind), Ethylenglycol, Ethylenglycolmonolaurat und andere Ester und Ether von Ethylenglycol oder Propylenglycol, 2-Octyldodecanol, Glycerin, Glycerinmonooleat, Glycerinmonostearat, hydriertes Rizinusöl, Isopropylmyristat, Isopropylpalmitat, Menthol oder andere flüchtige Terpenderivate (die Gemischbestandteile vieler natürlicher etherischer Öle sind), Methylbenzoat, Methyloctylsulfoxid, Mono- oder Diethylacetamid, N, N-Diethyl-m-toluamid, N-Methylpyrrolidon, Octanol-1 und andere flüchtige mittelkettige Alkohole, Octansäure und andere mittelkettige aliphatische Carbonsäuren, Oleylalkohol, Propandiol, Olivenöl, Ölsäure, Ölsäureoleylester, Phenylethanol, Propylenglycol, Rizinolsäure, Transcutol ®, Triacetin, aber auch Mischungen solcher Stoffe wie zum Beispiel Ölsäure/Propylenglycol oder Limonen/Dimethylisosorbid.

Als bevorzugte flüchtige und/oder thermolabile Hilfsstoffe seien die folgenden genannt: Cineol, Diethylenglycol, Dodecanol, Ethylenglycol, Propylenglycol, Menthol, Terpenderivat, N,N-Diethyl-m-toluamid, Propandiol und Transcutol®.

Zu den leichtflüchtigen und / oder thermolabilen Wirkstoffen zählen z.B. Nicotin, Nitroglycerin, Bupropion, Salicylsäure, Mecamylamin, Selegilin, Scopolamin, Venlafaxin, Oxybutynin, Benzatropin, Fenfluramin, Tulobuterol, Fentanyl, Sufentanil, Capsaicin, Methylsalicylat, Cyclopentamin, Ephedrin, ohne dass diese Auflistung erschöpfend wäre. Gemische dieser Stoffe, unabhängig vom Wirk- oder Hilfsstoffcharakter können in gleicher Weise eingesetzt werden und besonders vorteilhaft sein.

Über die vorstehende Eigenschaft der Flüchtigkeit hinaus besteht bei der Anwendung von trockener oder feuchter Hitze bei der Trocknung der transdermalen therapeutischen Systeme die Möglichkeit des vorzeitigen Abbaus von Wirk- oder Hilfsstoffen durch die Einwirkung von Hitze, Sauerstoff und Luftfeuchtigkeit bei hoher Konvektion der Trocknungsvehikel (Gase, insbesondere Luft).

Aus diesen Gründen sind klassische Verfahren der Herstellung transdermaler therapeutischer Systeme durchaus problematisch zu sehen und häufig nicht direkt anwendbar (s. z.B. "Dermatological Formulation and Transdermal Systems", Kenneth A. Walters and Keith R. Brain in Dermatological and Transdermal Formulations, New York 2002, Marcel Dekker, Seite 319-399, Herstellung insbesondere auf Seite 343 unten).

Es hat in der Vergangenheit nicht an Versuchen gefehlt, Herstellprozesse zur Vermeidung der genannten Substanzverluste durch Flüchtigkeit und / oder Thermolabilität zu entwickeln. So seien hier beispielhaft genannt die in DE 3629304, US 4,915,950 und insbesondere US 5,902,601 vorgeschlagenen Lösungen. In letzterer wird eine durch Zusatz von Polymeisko gemachte Zubereitung des Wirk- oder Hilfsstoffes auf ein Substrat exakt beschichtet und mit einer klebrigen wirkstofffreien Klebeschicht so laminiert, daß nach Migration des Wirk- oder Hilfsstoffes eine insgesamt verfestigte Matrix entsteht. Der Nachteil dieses Verfahrens nach dem Stande der Technik ist jedoch, wie auch Beispiel 2 der US 5,902,601 eingeräumt wird, die relativ langsame "Äquilibrierungszeit" (dort mit 60 min. genannt), die benötigt wird, bis das resultierende Produkt zu einem insgesamt scherfesten System wird. Da somit jede einzelne Stelle im herstellungstechnisch resultierenden Laminat im laufenden Produktionsprozeß nicht mechanisch belastet werden darf, läßt sich ein solcher Prozeß mit schwer diffusiblen Polymeren, wie z.B. Polyisobutylen, Styrol-Isopren-Copolymeren und selbst bei bestimmten Acrylatpolymeren kaum technisch umsetzen.

Aus der WO 95 / 24172 A ist ein Verfahren zum Herstellen einer Einheit zur transdermalen oder transmukosalen Arzneistoffabgabe bekannt, die eine Arzneistoff enthaltende, adhäsive, zusammengesetzte Schicht mit einem distalen Trägermaterial einschließt, das für den Arzneistoff undurchlässig ist und das auf die distale Oberfläche der zusammengesetzten Schicht laminiert ist, und mit einer proximalen abziehbaren Folie, welche für den Arzneistoff im Wesentlichen undurchlässig ist und so ausgebildet ist, dass sie entfernt werden kann, um den Arzneistoff an die Haut oder Schleimhaut zu verabreichen, und die auf die proximale Oberfläche der zusammengesetzten Schicht laminiert ist, wobei das Verfahren die folgenden Schritte umfasst:
a) Bereitstellen eines ersten adhäsiven Laminats, umfassend eine erste Arzneistoff durchlässige adhäsive Schicht, auf deren eine Oberfläche das distale Trägermaterial laminiert ist, wobei die zur ersten adhäsiven Schicht gegenüber liegende Oberfläche exponiert ist,
b) Bereitstellen eines zweiten adhäsiven Laminats, umfassend eine zweite adhäsive Schicht, auf deren eine Oberfläche die proximale abziehbare Folie laminiert ist, wobei die zur zweiten adhäsiven Schicht gegenüber liegende Oberfläche exponiert ist,
c) Extrudieren des Arzneistoffes in Gelform auf mindestens eine exponierte Oberfläche des ersten oder zweiten adhäsiven Laminats und
d) Zusammenlaminieren der exponierten Oberflächen des ersten adhäsiven Laminats und des zweiten adhäsiven Laminats, von welchen mindestens eine den extrudierten, gelierten Arzneistoff enthält, so dass die erste und die zweite adhäsive Schicht und der gelierte Arzneistoff so kombiniert sind, dass sie die Arzneistoff enthaltende, adhäsive, zusammengesetzte Schicht erzeugen, wobei das distale Trägermaterial deren eine Oberfläche bedeckt und wobei die proximale abziehbare Folie deren gegenüber liegende Oberfläche bedeckt.

US 5 462 746 A offenbart ein Pflaster für die transdermale Verabreichung von flüchtigen, pharmazeutisch wirksamen, chemisch basischen Inhaltsstoffen in Form eines Mehrfachelement-Systems, umfassend,
a) eine Matrix, in der als Wirkstoff physiologisch akzeptable Salze des flüchtigen Wirkstoffes verteilt sind, wobei die Matrix einen Haftkleber enthält,
b) eine haftklebende Zusammensetzung, die Aminogruppen enthält, zur Freisetzung der freien Base aus ihrem Salz,
c) eine für die diffundierbaren Inhaltsstoffe von (a) und (b) undurchlässige Rückschicht und
d) eine für die diffundierbaren Inhaltsstoffen von (a) und (b) undurchlässige Abziehfolie,
wobei die Matrix (a) oder zumindest ein Teil von (b), je nachdem, ob die Matrix (a) oder der Teil von (b) Kontakt mit der Abziehfolie (d) hat, eine Klebrigkeit (Tack) aufweist, die für die Befestigung des Pflasters auf der Haut ausreicht, und wobei jeder zwischen Matrix (a) und Abziehfolie (d) angebrachte Teil von (b) für den flüchtigen Wirkstoff oder eines seiner Salze oder für beide durchlässig ist.

In DE 43 32 094 C2 wird ein lösemittelfrei herstellbares Wirkstoffpflaster beschrieben, welches die nahezu verlustfreie Einarbeitung von der bei der üblichen Verarbeitungstemperatur flüchtigen Wirk- oder Hilfsstoffen ermöglicht. Das wird dadurch erreicht, daß eine erste Matrixschicht, welche bei der Herstellung eine streichfähige, molekularedisperse Lösung des Matrix Grundmaterials in dem leicht flüchtigen Wirk- oder Hilfsstoff als ausschließlichem Lösungsmittel darstellt, auf einen separat hergestellten Verbund aus einer oder mehreren weiteren Matrixschichten auflaminiert wird. Durch anschließende Migration des leichtflüchtigen Wirk- oder Hilfsstoffes in die angrenzende Matrix verliert sich die anfänglich streichfähige, dickflüssige Konsistenz der ersten Matrixschicht, und das Gesamtsystem wird weich-klebrig, aber formstabil bzw. scherstabil, wie es zur Verwendung als Wirkstoffpflaster erforderlich ist. Die Dauer dieses Vorgangs ("Reifungsprozeß") ist neben anderen physikalischen Parametern abhängig von den Diffusionseigenschaften aller Inhaltsstoffe sowie von der Gesamtgeometrie; sie beträgt wenige Minuten bis zu einigen Stunden, kann aber in einigen Fällen auch einige Tage bis zu einer Woche betragen.

Man erkennt, dass der unterschiedliche lange "Reifungsprozeß" zur Folge haben kann, dass ein kurze Zeit nach der Herstellung zur Verwendung kommendes TTS noch nicht die erforderliche Formstabilität erreicht hat. Zudem bedeutet das Verbleiben der ersten, nach der "Reifungszeit" nunmehr Wirkstoff- bzw. Hilfsstofffreien Matrixschicht in dem fertigen TTS eine zusätzliche Verdickung des Systems.

Aus den bekannten Stand der Technik ist ferner zu entnehmen, dass bei der Herstellung eines transdermalen therapeutischen Systems mit leicht flüchtigen und/oder thermolabilen Inhaltsstoffen nicht jede Kombination Matrixgrundpolymer/Inhaltsstoff möglich ist, sondern im Gegenteil die Kombinationsmöglichkeiten sehr begrenzt sind. So läßt sich z.B. ein Nicotin als (flüchtiger) Wirkstoff enthaltendes transdermales therapeutisches System nicht auf Basis einer Polyisobutylenmatrix herstellen, da das Nicotin auf die Matrix aufgebracht in Form einer flüssigen schwimmenden Schicht auf dieser verbleiben würde.

Die Aufgabe der vorliegenden Erfindung ist es daher, ein formstabiles transdermales therapeutisches System enthaltend flüchtige und/ oder thermolabile Wirk- und / oder Hilfsstoffe bereitzustellen, welches die Nachteile der aus dem Stand der Technik bekannten vergleichbaren Systeme vermeidet, insbesondere auch die Kombinationsmöglichkeiten Matrixgrundpolymer/flüchtige und/oder thermolabile Inhaltsstoffe erweitert.

Diese Aufgabe wird gelöst durch ein transdermales therapeutisches System gemäß Anspruch 1.

Das Aufeinanderlaminieren der Systemkomponenten kann in beliebiger Reihenfolge ausgeführt werden. So kann also die hautseitige polymere Matrixschicht (2), welche auch mehrschichtig sein kann (2',2") zunächst mit der Akzeptorschicht (4) und dann mit der Donatorschicht (3) laminiert werden. Es kann aber auch in umgekehrter Reihenfolge vorgegangen werden, beispielsweise kann auf einer hautseitig angeordneten Schicht (2) direkt auch Schicht (3), dann Schicht (4), und z.B. dann abschließend eine wirkstoffundurchlässige Rückschicht (1) folgen. Der erfindungsgemäße, überraschende Erfolg ergibt sich aus der Eigenschaft von Schicht (4), die flüchtige und / oder thermolabile Komponente aus Schicht (3) zügig zu übernehmen und dabei das System zunächst scherfest zu machen, bevor nach mehrstündiger Weitermigration ein großer Teil der flüchtigen und / oder thermolabilen Komponente, in die das schwerer diffusible Polymer enthaltende Schicht (3) hineindiffundiert ist. Der weitere Aufbau des empfindungsgemäßen Systems und die Komplettierung erfolgt mit den üblichen, dem Fachmann bekannten Komponenten und Techniken der Laminierung, des Wickelns, der Vereinzelung, Stanzung etc.

Üblicherweise werden mindestens noch eine in der Regel wirkstoffunlässige Rückschicht (Backing 1) hinzugefügt, ferner wird es, sofern nicht die Matrix (2) bereits klebrige Eigenschaften aufweist, eine Klebschicht zur Haut hin aufgebracht, sowie für den Zweck der Aufbewahrung des transdermalen therapeutischen Systems eine wiederablösbare Schutzschicht (Release Liner 5), die vor der Verwendung des Systems entfernt wird. Weiterhin könnte zum besseren Verbund der Donatorschicht bzw. je nach Aufbau der Akzeptorschicht mit der Rückschicht eine klebende Ankerschicht (6) vorhanden sein. Trotz der Mehrschichtigkeit weisen die erfindungsgemäßen Systeme anwendungstechnisch keinerlei Nachteile auf, da bei entsprechender Wahl der Schichtdicken die Systeme gleich dick oder sogar dünner auftragen als Systeme nach dem Stand der Technik. Vorteilhaft gegenüber dem Stand der Technik ist die bei gegebenem Schutz vor temperaturbedingter Flüchtigkeit oder Instabilität der Komponenten gegebene ausgesprochen beschleunigte Prozeßfähigkeit, welche die Herstellbarkeit solcher Systeme im industriellen Maßstab zur Folge hat.

Weiterhin werden insbesondere klebende Polymere vor der Formulierungstechnik erschlossen, die aufgrund ihres trägen Wirkstoff- und Hilfsstoffpermeationsverhaltens herstellungstechnisch bisher für Migrationsverfahren nicht in Frage kamen. Zu solchen Polymeren zählen insbesondere die bei transdermalen Systemen sehr üblichen Polymergruppen:
Styrol-Isopren-Copolymere, Polyisobutylene, Acrylatcopolymere, Butylkautschuk, Polybutylene und einige andere vergleichbare Polymere mit deutlich geringerem Aufnahmepotential.

Die Natur der flüchtigen und / oder thermolabilen Komponenten und die in Frage kommenden Kandidaten wurden einleitend bereits beschrieben. Basispolymere für die Matrixschicht (2) können sein: Polyisobutylen, Polybutylen, Styrol-Isopren, Styrol-Copolymer, Styrol-Butadien-Styrol-Blockcöpolymer: Dabei können weitere Komponenten wir Harze, Öle, Füllstoffe und andere pharmazeutisch übliche Modifikatoren benutzt werden.

Bevorzugt kommen als Basispolymere für die Matrixschicht(en) Polyisobutylen, Polybutylen und Butylkautschuk, insbesondere Polyisobutylen und Polybutylen in Frage.

Die Schicht kann durch klassische Techniken des Lösens, Mischens, Beschichtens und temperaturgeschützten Trocknens erzeugt werden, da in ihr die flüchtige und / oder thermolabile Komponente noch nicht enthalten ist. Gleiches gilt für die Akzeptorschicht (4), die jedoch nicht aus schwer diffusiblen Komponenten besteht, sondern aus Polymeren mit deutlich beschleunigtem Aufnahmeverhalten für Wirk- und Hilfsstoffe. Die Schicht kann klebrig oder nichtklebrig eingestellt werden. Beispielhafte Grundpolymere können hier daher auch sowohl wasser- wie auch organisch polymerlöslich angesetzt sein. Beispielhaft seien Stoffe wie Polyvinylalkohol, Polyvinylacetat, Siliconklebemassen und Siliconkautschuke, Acrylat- und Methylacrylat-Copolymere genannt.

Es ist jedoch durchaus auch möglich, schwerer diffusible Polymere und Zusatzstoffe zu verwenden, sofern immer noch gegenüber Schicht (2) ein beschleunigtes Wirkstoffaufnahmeverhalten resultiert. In einer besonderen Ausführungsform kann dies auch durch Einbetten von Partikeln stark quellender Stoffe (PVP, Polyvinylalkohol) in einer Grundmatrix aus schwer diffusiblen Polymeren erfolgen.

### Beispiele:

### Beispiel 1:

a) Eine Lösung aus einem Gemisch aus niedermolekularem und hochmolekularem Polyisobutylen, Polybutylen und einem aliphatischen Kohlenwasserstoff wird so auf eine intermediäre siliconisierte 36µm PET-Folie aufgetragen (Ankerschicht (6)), dass nach dem Trocknen ein Auftragsgewicht von ca. 110 g/m² resultiert. Dieser Kleberstrich wird nach dem Trocknen mit einer transparenten 23 µm PET-Folie (1) abgedeckt.
b) In einem zweiten Arbeitsschritt wird die gleiche Polymerlösung aus (a) so auf eine siliconisierte 100 µm PET-Folie (5) aufgetragen, dass nach dem Trocknen ein Kleberstrich von ca. 110 g/m² (2) resultiert. Dieser Kleberstrich wird mit der siliconisierten Seite einer 36 µm PET-Folie abgedeckt.
c) Anschließend wird eine Lösung aus Poly(butylmethacrylat) und Essigsäureethylester so auf eine intermediäre unsiliconisierte 36 µm PET-Folie beschichtet, dass nach dem Trocknen ein Auftragsgewicht von ca. 90 g/m² resultiert (4). Unmittelbar nach dem Trocknen wird der Film aus Poly(butylmethacrylat, methylenacrylat) auf den Kleberstrich aus (b) kaschiert (die 36 µm PET-Folie wird vorher abgezogen).
d) Danach wird eine Lösung aus Nicotin und Poly(butylmethacrylat, methylenacrylat) so auf den Poly(butylmethacrylat, methylenacrylat)-Film beschichtet, dass ein Auftragsgewicht von ca. 60 g/m² resultiert (3). Zum Schluß wird die Ankerschicht aus (a) nach Abziehen der 36 µm PET-Folie auf den Verbund aus 100 µm PET-Folie (5), Matrix (2), Poly(butylmethacrylat, Methylenacrylat) (4) und Nicotin/Poly(butylmethacrylat, methylenacrylat) (3) kaschiert. Es resultiert das fertige Nicotin-Laminat. In entsprechender Weise werden die transdermalen therapeutischen Systeme gemäß den Varianten 1-4 hergestellt.

| **Beispiel 1 (Fig. C)** | | **Variante 1 (Fig. A)** | | **Variante 2 (Fig. B)** | | **Variante 3 (Fig. D)** | | **Variante 4 (Fig. E)** | |
|---|---|---|---|---|---|---|---|---|---|
| **1** | PET-Folie, 23µm transp. | **1** | PET-Folie, 23µm transp. | **1** | PET-Folie, 23µm transp. | **1** | PET-Folie, 23µm transp. | **1** | PET-Folie, 23µm transp. |
| **6** | PIB/PB-Matrix, ca. 110g/m² | **3** | Nic/Plastoid B, 1,4:1 ca. 60g/m² | **4** | Plastoid B, ca. 90g/m² | **6** | PIB/PB-Matrix, ca. 110g/m² | **6** | PIB/PB-Matrix, ca. 110g/m² |
| **3** | Nic/Plastoid B, ca. 1,4:1 ca. 60g/m² | **4** | Plastoid B, ca. 90g/m² | **3** | Nic/Plastoid B, 1,4:1 ca. 60g/m² | **4** | Plastoid B, ca. 90g/m² | **3** | Nic/Plastoid B, 1,4:1 ca. 60g/m² |
| **4** | Plastoid B, ca. 90g/m² | **2** | PIB/PB-Matrix, ca. 110g/m² | **2** | PIB/PB-Matrix, ca. 110g/m² | **3** | Nic/Plastoid B, 1,4:1 ca. 60g/m² | **4** | Plastoid B, ca. 90g/m² |
| **2** | PIB/PB-Matrix, ca. 110g/m² | **5** | PET-Folie 100µm transp. | 5 | PET-Folie 100µm transp. | **2** | PIB/PB-Matrix, ca. 110g/m². | **2** | PIB/PB-Matrix, ca. 110g/m² |
| **5.** | PET-Folie 100µm transp. | | | | | **5** | PET-Folie 100µm transp | **(2'2")** | GSM 3083, ca. 40g/m² |
| | | | | | | | | **5** | PET-Folie 100µm transp. |

| | | | | | | | | | |
|---|---|---|---|---|---|---|---|---|---|
| 1 = Backing 2 = Matrix 2'2" = Optional mehrschichtige Matrix 3 = Donator 4 = Akzeptor 5 = Release Liner 6 = Optionale Ankerschicht | | | | | | | | | |

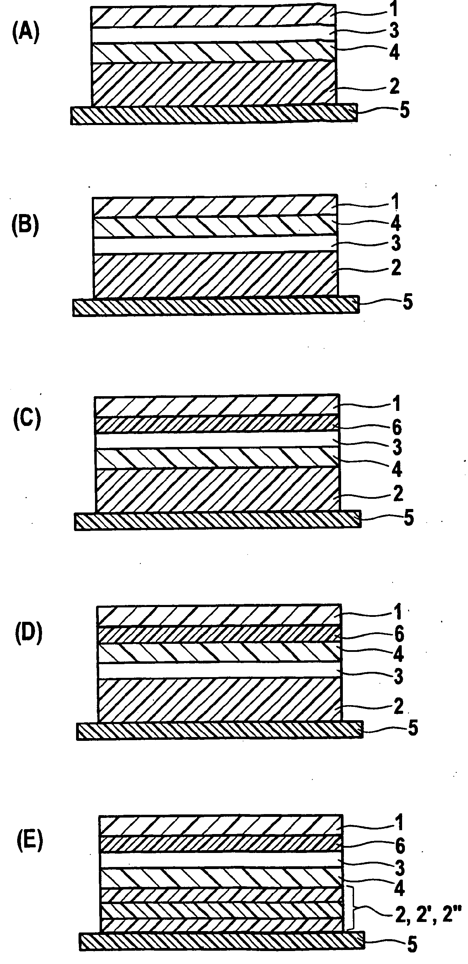

## Patentansprüche

1. Transdermales therapeutisches System enthaltend Nicotin und/oder Hilfsstoff, bestehend aus einer Rückschicht, welche für Nikotin und den Hilfsstoff im wesentlichen undurchlässig ist, mindestens zwei, auf die Rückschicht folgende polymere Matrixschichten, von denen mindestens eine als Akzeptorschicht dient, welche Nikotin und/oder den Hilfsstoff enthält, und einer wiederablösbaren Schutzschicht, **dadurch gekennzeichnet, dass** das genannte transdermale therapeutische System herstellbar ist durch Aufeinanderlaminieren der folgenden aneinandergrenzenden Schichten:
a) einer im wesentlichen wirkstaffundurchlässigen Rückschicht,
b) ggf. einer als Ankerschicht dienenden Matrixschicht,
c) einer zum Zeitpunkt der Herstellung des transdermalen therapeutischen Systems Nikotin enthaltene, als Donatorschicht dienende, polymere Matrixschicht;
d) eine als Akzeptorschicht dienende, polymere Matrixschicht,
e) einer weiteren hautseitig angeordneten polymeren Matrixschicht,
f) einer wiederablösbaren Schutzschicht,
wobei die Akzeptorschicht die Eigenschaft besitzt, Nikotin und/oder Hilfsstoff beschleunigt aufzunehmen und dabei das System scherfest macht, wobei für Donator- und Akzeptorschicht das gleiche Basispolymer verwendet wird, und wobei während des Laminierens oder kurze Zeit nach dem Laminieren die Donatorschicht sich durch Migration des Nikotins und/oder Hilfsstoffe in die Akzeptorschicht mit der letzteren vereinigt.

2. Transdermales therapeutisches System gemäß Anspruch 1, **dadurch gekennzeichnet,dass** die als Ankerschicht dienende Matrixschicht haftklebend mit der Rückschicht verbunden ist.

3. Transdermales therapeutisches System gemäß mindestens einem der vorhergehenden Ansprüche, **dadurch gekennzeichnet, dass** als Basispolymere für Matrixschicht(en), mit Ausnahme von Donator- und Akzeptorschicht, Polyisobutylen, Polybutylen, Styrol-Isopren, Styrol-Copolymere und/oder Styrol-Butadien-Styrol-Blockpolymere verwendet werden.

4. Transdermales therapeutisches System gemäß Anspruch 3, **dadurch gekennzeichnet, dass** als Basispolymere Polyisobutylen und/oder Polybutylen verwendet werden.

5. Transdermales therapeutisches System gemäß Anspruch 1, **dadurch gekennzeichnet, dass** als Basispolymer für Donator- und Akzeptorschicht Polyvinylalkohol, Polyvinylacetat, Silikonkautschuke, Acrylat-Copolymere, oder Methylacrylat-Copolymere verwendet werden.

6. Transdermales therapeutisches System gemäß Anspruch 1, **dadurch gekennzeichnet, dass** als Basispolymer für Donator- und Akzeptorschicht ein neutrales Copolymer basierend auf Butylmethacrylat und Methylmcthacrylat verwendet wird.

7. Transdermales therapeutisches System gemäß mindestens einem der vorhergehenden Ansprüche **dadurch gekennzeichnet, dass** die hautseitig angeordnete Matrixschicht Klebeeigenschaften besitzt.

8. Transdermales therapeutisches System gemäß mindestens einem der vorhergehenden Ansprüche **dadurch gekennzeichnet, dass** die hautseitig angeordnete Matrixschicht mit einer Haftkleber-Schicht versehen ist.

9. Verfahren zur Herstellung eines transdermalen therapeutischen Systems gemäß Anspruch 1, **dadurch gekennzeichnet, dass** zunächst durch Beschichtung und Trocknung eine für Nikotin und/oder Hilfsstoff geeignete Akzeptozschicht hergestellt wird, auf diese Nikotin und/oder Hilfsstoff entweder allein oder in Mischung mit weiteren Hilfsstoffen als Donatorschicht aufgebracht wird, die auf diese Weise beschichtete Akzeptorschicht unmittelbar mit einer Rückschicht (Backing) abgedeckt oder zunächst mit einer Ankerschicht verbunden und dann mit einer Rückschicht abgedeckt wird und das so hergestellte Laminat mit mindestens einer auf den gegenüberliegenden Seite mit einer wiederablösbaren Schicht (Release Liner) versehenen Matrix vereinigt.

## Claims

1. A Transdermal therapeutic system that contains nicotine and/or an excipient and that consists of a backing that is essentially impervious to nicotine and the excipient, at least two polymer matrix layers following the backing, at least one of which serves as an acceptor layer that contains nicotine and/or the excipient, and a release liner, **characterized in that** the mentioned transdermal therapeutic system can be produced by laminating the following adjacent layers onto one another:
a) a backing that is essentially impervious to the active ingredient;
b) possibly a matrix layer serving as an anchor layer;
c) a polymer matrix layer that contains nicotine at the time when the transdermal therapeutic system is produced and that serves as a donor layer;
d) a polymer matrix layer serving as an acceptor layer;
e) another polymer matrix layer arranged on the skin side;
f) a release liner,
the acceptor layer having the property of absorbing nicotine and/or the excipient at an accelerated rate and giving the system shear strength, the same base polymer being used for the donor and acceptor layers, and the donor layer uniting with the acceptor layer due to the migration of the nicotine and/or excipients into the acceptor layer during or shortly after the lamination.

2. The transdermal therapeutic system described in claim 1, **characterized in that** the matrix layer serving as the anchor layer is adhesively connected with the backing.

3. The transdermal therapeutic system described in at least one of the preceding claims, **characterized in that** polyisobutylene, polybutylene, styrene-isoprene, styrene copolymers, and/or styrene-butadiene-styrene block polymers are used as the base polymers for the matrix layer(s), with the exception of the donor and acceptor layer.

4. The transdermal therapeutic system described in claim 3, **characterized in that** polyisobutylene and/or polybutylene are used as the base polymers.

5. The transdermal therapeutic system described in claim 1, **characterized in that** polyvinyl alcohol, polyvinyl acetate, silicone rubbers, acrylate copolymers, or methyl acrylate copolymers are used as the base polymer for the donor and acceptor layers.

6. The transdermal therapeutic system described in claim 1, **characterized in that** a neutral copolymer based on butyl methacrylate and methyl methacrylate is used as the base polymer for the donor and acceptor layers.

7. The transdermal therapeutic system described in at least one of the preceding claims, **characterized in that** the matrix layer arranged on the skin side has adhesive properties.

8. The transdermal therapeutic system described in at least one of the preceding claims, **characterized in that** the matrix layer arranged on the skin side has a pressure-sensitive adhesive layer.

9. A process for producing the transdermal therapeutic system described in claim 1, **characterized by** first producing an acceptor layer suitable for nicotine and/or an excipient by coating and drying, applying nicotine and/or excipient onto it, either alone or mixed with other excipients, to form a donor layer, covering the acceptor layer coated in this way with a backing, [either] directly or first connecting it with an anchor layer and then covering it with a backing, and uniting the laminate produced in this way with at least one matrix that has a release liner on the opposite side.

## Revendications

1. Système thérapeutique transdermique contenant de la nicotine et/ou une substance auxiliaire, constitué d'une couche arrière, laquelle est essentiellement imperméable à la nicotine et à la substance auxiliaire, d'au moins deux couches de matrices polymères succédant à la couche arrière, parmi lesquelles au moins une sert de couche acceptrice, laquelle contient la nicotine et/ou la substance auxiliaire, et d'une couche de protection pouvant de nouveau être détachée, **caractérisé en ce que** ledit système thérapeutique transdermique peut être fabriqué par une stratification des couches voisines les unes des autres suivantes :
a) une couche arrière essentiellement imperméable à la matière active,
b) éventuellement, une couche de matrice servant de couche d'ancrage,
c) une couche de matrice polymère contenant de la nicotine au moment de la fabrication du système thérapeutique transdermique, servant de couche donneuse ;
d) une couche de matrice polymère servant de couche acceptrice,
e) une autre couche de matrice polymère disposée du côté de la peau,
f) une couche de protection pouvant de nouveau être détachée,
où la couche acceptrice possède la propriété d'absorber de manière accélérée la nicotine et/ou la substance auxiliaire et rendre ainsi le système résistant au cisaillement, où le même polymère de base est employé pour les couches donneuse et acceptrice, et où, pendant la stratification, ou peu de temps après la stratification, la couche donneuse s'unit avec la couche acceptrice par une migration de la nicotine et/ou de la substance auxiliaire dans cette dernière.

2. Système thérapeutique transdermique selon la revendication 1, **caractérisé en ce que** la couche de matrice servant de couche d'ancrage est reliée à la couche arrière de manière auto-adhésive.

3. Système thérapeutique transdermique selon au moins l'une des revendications précédentes, **caractérisé en ce qu'**en tant que polymères de base pour la(les) couche(s) de matrice, à l'exception des couches donneuse et acceptrice, du polyisobutylène, du polybutylène, de styrène-isoprène, des copolymères de styrène et/ou des polymères séquencés styrène - butadiène - styrène sont employés.

4. Système thérapeutique transdermique selon la revendication 3, **caractérisé en ce qu'**en tant que polymères de base du polyisobutylène et/ou du polybutylène sont employés.

5. Système thérapeutique transdermique selon la revendication 1, **caractérisé en ce qu'**en tant que polymère de base pour les couches donneuse et acceptrice, de l'alcool polyvinylique, de l'acétate de polyvinyle, des caoutchoucs de silicone, des copolymères acrylate ou des copolymères méthylacrylate sont employés.

6. Système thérapeutique transdermique selon la revendication 1, **caractérisé en ce qu'**en tant que polymère de base pour les couches donneuse et acceptrice, un copolymère neutre basé sur du méthacrylate de butyle et du méthacrylate de méthyle est employé.

7. Système thérapeutique transdermique selon au moins l'une des revendications précédentes, **caractérisé en ce que** la couche de matrice disposée du côté de la peau possède des propriétés adhésives.

8. Système thérapeutique transdermique selon au moins l'une des revendications précédentes, **caractérisé en ce que** la couche de matrice disposée du côté de la peau est pourvue d'une couche d'adhésif sensible à la pression.

9. Procédé de fabrication d'un système thérapeutique transdermique selon la revendication 1, **caractérisé en ce que** d'abord une couche acceptrice appropriée pour de la nicotine et/ou une substance auxiliaire est fabriquée par un revêtement et un séchage, sur laquelle de la nicotine et/ou une substance auxiliaire sont rapportées soit seules, soit sous forme d'un mélange avec d'autres substances auxiliaires servant de couche donneuse, la couche acceptrice qui est revêtue immédiatement de cette manière d'une couche arrière (backing) ou d'abord reliée avec une couche d'ancrage et ensuite revêtue avec une couche arrière et le stratifié ainsi fabriqué s'assemble avec au moins une matrice munie d'une couche pouvant être de nouveau détachée (bande auto-adhésive) située sur le côté en face.
